# FASCICULE DE BREVET EUROPEEN

(11) **EP 4 415 658 B1**
(45) Date de publication et mention de la délivrance du brevet: **13.08.2025**
(21) Numéro de dépôt: 22802203.4
(22) Date de dépôt: 10.10.2022
(51) Int. Cl.: A61F 2/66, A61F 2/54, A61F 2/50

(54) **ENSEMBLE ARTICULÉ**
GEGLIEDERTE BAUGRUPPE
ARTICULATED SET

(30) Priorité: 13.10.2021 FR 2110831
(43) Date de publication de la demande: 21.08.2024
(73) Titulaire: Revival Bionics, 60280 Venette (FR)
(72) Inventeur: BANIEL, Guillaume, Roger, Albert, Patrick, 60500 CHANTILLY (FR)
(74) Mandataire: Ernest Gutmann - Yves Plasseraud S.A.S.
(86) Numéro de dépôt international: PCT/FR2022/051902
(87) Numéro de publication internationale: WO 2023/062308

(56) Documents cités:
- EP-A1- 0 413 450
- EP-B1- 1 718 252
- WO-A1-97/12567
- DE-C- 817 186

## Description

### Domaine technique

La présente divulgation concerne le domaine des ensembles articulés utilisés dans des prothèses actives ou passives. Plus particulièrement, la présente divulgation concerne le domaine des prothèses de pieds. EP 1 718 252 B1 est regardé comme l'état de la technique le plus proche.

Les prothèses, actives ou passives, ont pour objectif de compenser le handicap des personnes amputées en assurant la ou les fonctions du membre amputé. Par exemple, en fonction du degré d'amputation, une prothèse de pied se doit d'assurer potentiellement la fonction du mollet, des muscles releveurs du pied, ou encore la fonction relative au tendon d'Achille. En outre, les prothèses, en particulier les prothèses actives, se doivent aussi d'intégrer un contrôleur et une batterie afin de remplacer la fonction de contrôle normalement assurée par le cerveau et de compenser la dépense énergétique normalement assurée par le métabolisme.

Ainsi, les prothèses actives comportent généralement un actionneur électrique linéaire composé d'un groupe moteur-vis. Cet actionneur est généralement mis en série avec un élément ressort faisant office de tendon d'Achille artificiel et permettant de diminuer le pic de puissance à fournir par l'actionneur lors de l'utilisation, le couple actionneur/ressort étant connu sous le nom « Series Elastic Actuator ».

De nombreuses solutions ont été proposées par l'art antérieur pour répondre aux besoins de personnes impactées par une amputation, en particulier le besoin de confort, de compacité, d'autonomie de la prothèse et d'adaptabilité aux caractéristiques de l'utilisateur. Cependant, les solutions proposées de l'art antérieur ne permettent généralement pas d'adresser correctement les besoins précités et de nombreux inconvénients demeurent. Par exemple, dans le cas des prothèses de pied, les ressorts constituant le tendon d'Achille artificiel n'ont pas toujours une raideur adaptée spécifiquement à un patient ou sont difficilement adaptables à la masse de chaque patient.

En outre, les choix architecturaux des solutions de l'art antérieur peuvent limiter l'intégration de l'ensemble des composants nécessaires à la réalisation d'une prothèse active (comprenant par exemple un moteur, de l'électronique, une batterie, un tendon d'Achille artificiel tel qu'un ressort) dans une enveloppe de conception raisonnablement identique au volume du membre absent, ou peuvent encore limiter l'intégration d'un nombre de cellules de batteries compatibles avec une autonomie d'une journée ou plus, au bénéfice des patients.

La présente divulgation vient améliorer la situation.

Il est ainsi proposé un ensemble articulé pouvant comprendre :
- un élément d'entrée,
- un élément de sortie,
- une barre de torsion apte à se déformer autour d'un axe longitudinal de la barre de torsion,
- des moyens d'actionnement,
dans lequel l'élément d'entrée est couplé en rotation à une première zone de la barre de torsion et l'élément de sortie est couplé en rotation à une deuxième zone de la barre de torsion, écartée de ladite première zone, l'élément d'entrée étant en outre relié aux moyens d'actionnement, l'élément d'entrée et l'élément de sortie étant guidés en rotation l'un par rapport l'autre suivant un axe de la barre de torsion.

De manière avantageuse, un tel ensemble articulé permet d'obtenir un système d'accumulation et de restitution d'énergie compact, au travers de la barre de torsion, en particulier lorsqu'il est utilisé au sein d'un appareil prothétique ou orthétique. Une telle structure permet de réduire l'encombrement de l'ensemble, de façon à pouvoir intégrer plus facilement d'autres éléments, tels qu'une ou plusieurs batteries, des circuits, un ou plusieurs moteurs, etc.

L'élément d'entrée et l'élément de sortie peuvent être reliés respectivement à la première zone et à la deuxième zone de la barre de torsion par l'intermédiaire de liaisons encastrement.

La barre de torsion peut présenter une valeur de raideur comprise entre 100 et 10 000 N.m par radian, de préférence entre 100 et 2 000 N.m par radian.

La barre de torsion, l'élément d'entrée et l'élément de sortie peuvent être configurés de manière à ce que la barre de torsion soit amovible.

Ainsi, la barre de torsion peut être facilement remplacée lorsque celle-ci est abimée ou lorsqu'il est nécessaire d'adapter la raideur de la barre de torsion. Avantageusement, lorsque l'ensemble articulé est intégré dans une prothèse (e.g. active), il est possible d'adapter la raideur de la barre de torsion en fonction de la morphologie ou physionomie du patient. Par exemple, il est possible de disposer de classes de barres de torsion, où chaque classe est adaptée à une physionomie d'un patient, comme par exemple sa masse.

L'ensemble peut comprendre une embase articulée par rapport à l'élément d'entrée, l'embase supportant les moyens d'actionnement.

Les moyens d'actionnement peuvent comprendre un actionneur linéaire configuré pour déplacer un organe d'actionnement apte à être déplacé selon un axe d'actionnement non colinéaire, par exemple perpendiculaire, à l'axe de la barre de torsion, le déplacement de l'organe d'actionnement entrainant la rotation de l'élément d'entrée par rapport à l'élément de sortie.

L'actionneur linéaire peut être un vérin hydraulique, un vérin pneumatique, un système vis-écrou entrainé par un moteur, par exemple de type vis à billes ou vis à rouleaux planétaires ou nommés également vis à rouleaux satellites, un moteur électrique, etc. Ainsi, selon un exemple, lorsque l'actionneur linéaire est un système vis-écrou, l'organe d'actionnement peut correspondre à une vis mise en mouvement par l'écrou entrainé par le moteur. Selon un autre exemple, lorsque l'actionneur linéaire est un système vis-écrou, l'organe d'actionnement peut correspondre à un écrou mis en mouvement par une vis entrainée par le moteur.

Le système vis-écrou entrainé par un moteur peut comprendre une poulie menante couplée au moteur et une poulie menée couplée à la vis, la poulie menante et la poulie menée étant relié par une courroie d'entrainement.

La puissance maximale fournie par l'actionneur peut être comprise entre 50 et 1000 Watts, dans le cas d'une utilisation pour une prothèse et lors de la marche.

Le volume total de l'actionneur et/ou le volume total du moteur avec un système vis-écrou peut être compris entre 100 et 1500 cm³.

L'organe d'actionnement peut être en liaison pivot ou rotule avec l'élément d'entrée.

La liaison pivot entre l'organe d'actionnement et l'élément d'entrée peut être réalisée à travers un palier, par exemple un palier lisse, un roulement (à billes, à rouleaux ou à aiguilles), un embout à rotule. L'axe de pivot peut être fixé par frettage ou par anneaux élastiques fendus.

Les moyens d'actionnement peuvent être montés de manière directe ou indirecte sur ou dans l'embase. Par indirecte, il peut être entendu qu'un élément additionnel est agencé entre les moyens d'actionnement et l'embase.

Des moyens de contrôle, tels qu'une unité de contrôle, peuvent permettre de piloter les différents éléments de l'appareil prothétique ou orthétique, ces moyens de contrôle peuvent être montés sur et/ou dans l'embase. Les moyens de contrôle peuvent être également montés sur l'ensemble d'actionnement

L'actionneur linéaire peut être articulé à l'embase selon une liaison pivot dont l'axe de pivot est parallèle à l'axe de la barre de torsion et dans lequel la torsion de la barre de torsion ou la rotation de l'élément d'entrée entraine la rotation de l'actionneur linéaire par rapport à l'embase autour de l'axe de pivot.

Dans un ou plusieurs modes de réalisation, au moins un ressort peut être compris entre l'actionneur linéaire et l'embase, l'au moins un ressort peut être configuré pour se comprimer ou se détendre lors du mouvement relatif entre l'actionneur linéaire et ladite embase.

Avantageusement, il est alors possible d'ajuster une valeur de raideur globale de l'ensemble articulé au niveau de la barre de torsion., Dans un ou plusieurs modes de réalisation, l'au moins un ressort peut être configuré de façon à ce que la raideur globale au niveau de la barre de torsion peut être comprise entre 100 et 10 000 N.m par radian.

Avantageusement, la prise en compte de tous les composants dans le calcul de raideur équivalente du système ramenée à l'axe de la barre de torsion permet la réduction du pic de puissance au niveau du moteur.

Par ailleurs, le fait que plusieurs composants participent, en série, à la raideur globale permet d'ajuster précisément la raideur de la barre de torsion ainsi que sa tenue en fatigue.

Dans un ou plusieurs modes de réalisation, une première extrémité de la barre de torsion forme la première zone et une deuxième extrémité de la barre de torsion forme la deuxième zone, ladite barre de torsion étant configurée pour se déformer par torsion dans une portion de travail comprise entre la première zone et la deuxième zone,
l'élément d'entrée et l'élément de sortie comprenant respectivement une première section tubulaire et une deuxième section tubulaire configurées pour s'emboîter l'une dans l'autre en formant mutuellement un corps tubulaire logeant la barre de torsion, la première section tubulaire de l'élément d'entrée comprenant une première surface de couplage intérieure à une première extrémité du corps tubulaire configurée pour être couplée en rotation avec la première zone de la barre de torsion autour de l'axe de la barre de torsion et la deuxième section tubulaire de l'élément de sortie comprenant une deuxième surface de couplage intérieure à une deuxième extrémité du corps tubulaire configurée pour être couplée en rotation avec la deuxième zone de la barre de torsion, autour de l'axe de la barre de torsion et dans lequel la première section tubulaire et la deuxième section tubulaire comprennent respectivement des premières surfaces de guidage, respectivement intérieures et extérieures à la première section tubulaire et à la deuxième section tubulaire, ou inversement , les premières surfaces de guidage étant agencées en une position intermédiaire entre la première extrémité et la deuxième extrémité du corps tubulaire, les premières surfaces de guidage intérieures et extérieures étant configurées pour assurer un guidage en rotation de l'élément de sortie par rapport à l'élément d'entrée autour de l'axe de la barre de torsion.

Par deuxième extrémité du corps tubulaire, il peut être entendu une deuxième extrémité opposée à la première extrémité du corps tubulaire.

Les surfaces de guidage peuvent assurer le guidage par contact direct ou peuvent être réalisées par l'intermédiaire d'un palier, comme par exemple une bague, un coussinet ou un roulement.

L'élément de sortie peut comprendre une deuxième partie, distincte de la deuxième section tubulaire, en liaison pivot avec la première section tubulaire de l'élément d'entrée, par des deuxièmes surfaces de guidage respectivement extérieures et intérieures à la première section tubulaire et à la deuxième partie, agencées à la première extrémité du corps tubulaire.

Le corps tubulaire s'étendant entre la première extrémité et la deuxième extrémité peut loger entièrement la barre de torsion, la barre de torsion étant de préférence de même longueur que le corps tubulaire suivant l'axe de la barre de torsion.

Ainsi, de manière avantageuse, il est possible d'obtenir un système d'accumulation et de restitution d'énergie très compacte, idéal pour de nombreux dispositifs tels que les grands appareils orthopédiques (prothèses et orthèses), ou encore les dispositifs robotiques (e.g. cobot ou exosquelette).

La présente divulgation porte aussi sur un appareil prothétique selon la présente divulgation, l'appareil prothétique pouvant comprendre :
- l'embase est une embase tibiale destinée à être fixée à un moignon, par exemple à travers une emboiture, l'embase tibiale embarquant les moyens d'actionnement,
- une lame d'appui au sol configurée pour pivoter par rapport à l'embase autour de l'axe de la barre de torsion,

et dans lequel l'élément d'entrée est connecté à une sortie des moyens d'actionnement et l'élément de sortie est relié à la lame d'appui au sol,
la barre de torsion étant configurée, lors de la marche, pour emmagasiner une énergie par torsion de la barre de torsion, et la restituer pour la propulsion simultanément au travail des moyens d'actionnement.

Une telle structure permet de reproduire plus fidèlement les caractéristiques biomécaniques d'une cheville humaine et notamment son caractère propulsif.

L'embase tibiale peut comprendre un corps creux recevant les moyens d'actionnement.

De manière avantageuse, cette embase peut protéger les moyens d'actionnement, faire l'objet d'un travail de conception permettant de soigner l'esthétique du système au bénéfice des patients.

Le corps creux de l'embase tibiale peut également embarquer des moyens de contrôle tels qu'une unité de contrôle permettant de piloter les différents éléments de l'appareil prothétique.

L'embase tibiale peut présenter une direction longitudinale perpendiculairement à la barre de torsion, les moyens d'actionnement comprenant un moteur et un système vis-écrou, le système vis-écrou et le moteur étant agencés en recouvrement suivant l'axe longitudinal.

De manière avantageuse, un tel agencement permet d'augmenter la compacité de l'appareil prothétique, de limiter sa hauteur afin d'adresser une quantité plus importante de patients dont la longueur de moignon et la taille sont variables, sans sacrifier les performances requises pour les moyens d'actionnement.

Le système vis-écrou entrainé par un moteur peut comprendre une poulie menante couplée au moteur et une poulie menée couplée à la vis, la poulie menante et la poulie menée étant relié par une courroie d'entrainement.

Un espace peut être ménagé entre la barre de torsion et la plaque d'appui, de manière à recevoir une source d'énergie autonome, par exemple amovible, pour les moyens d'actionnement.

Ainsi de manière avantageuse, il est possible de diminuer l'encombrement total de l'appareil prothétique en évitant que la batterie soit disposée sur l'extérieur de l'appareil. De plus, la batterie peut ainsi être dissimulée dans une enveloppe, par exemple une enveloppe esthétique en forme de pied, destinée à être chaussée.

La présente divulgation porte aussi sur un grand appareil orthopédique comportant au moins un ensemble articulé selon la présente divulgation.

La présente divulgation concerne également un système articulé comprenant au moins un ensemble articulé du type précité, dans lequel le système articulé comprend au moins une première section et une deuxième section articulées l'une par rapport l'autre, la première section formant l'élément d'entrée de l'ensemble articulé, la deuxième section formant l'élément de sortie de l'ensemble articulé.

### Brève description des dessins

D'autres caractéristiques, détails et avantages apparaîtront à la lecture de la description détaillée ci-après, et à l'analyse des dessins annexés, sur lesquels :
[Fig. 1a] illustre schématiquement une vue en perspective de l'ensemble articulé de la présente divulgation.
[Fig. 1b] illustre schématiquement une vue éclatée de l'ensemble articulé de la présente divulgation.
[Fig. 1c] illustre schématiquement une vue en coupe suivant le plan ZY de l'ensemble articulé de la présente divulgation.
[Fig. 2a] illustre schématiquement une vue en perspective d'un appareil prothétique telle qu'une prothèse de pied comprenant l'ensemble articulé de la présente divulgation.
[Fig. 2b] illustre schématiquement une vue de côté suivant le plan ZX d'un appareil prothétique tel qu'une prothèse de pied comprenant l'ensemble articulé de la présente divulgation.
[Fig. 2c] illustre schématiquement une vue en coupe suivant le plan ZY d'un appareil prothétique tel qu'une prothèse de pied comprenant l'ensemble articulé de la présente divulgation.

### Description des modes de réalisation

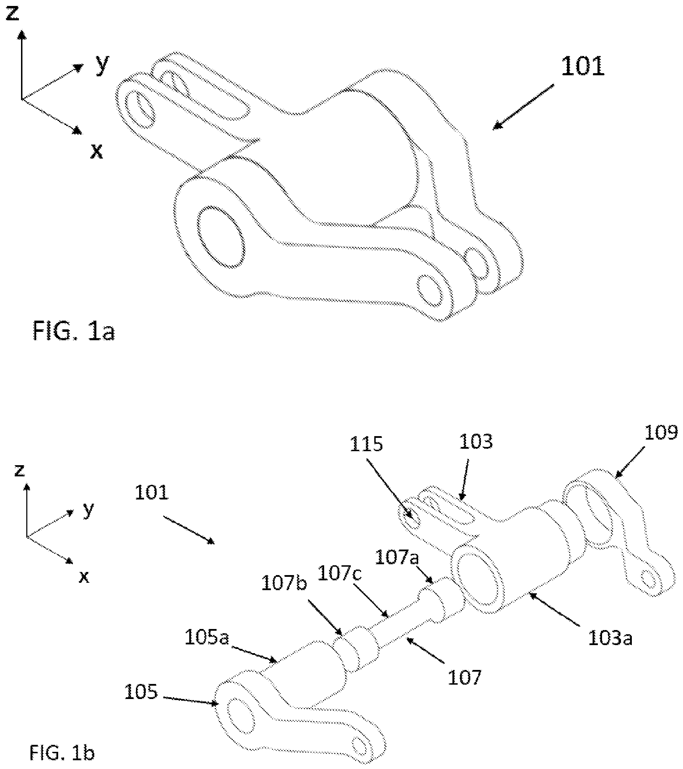

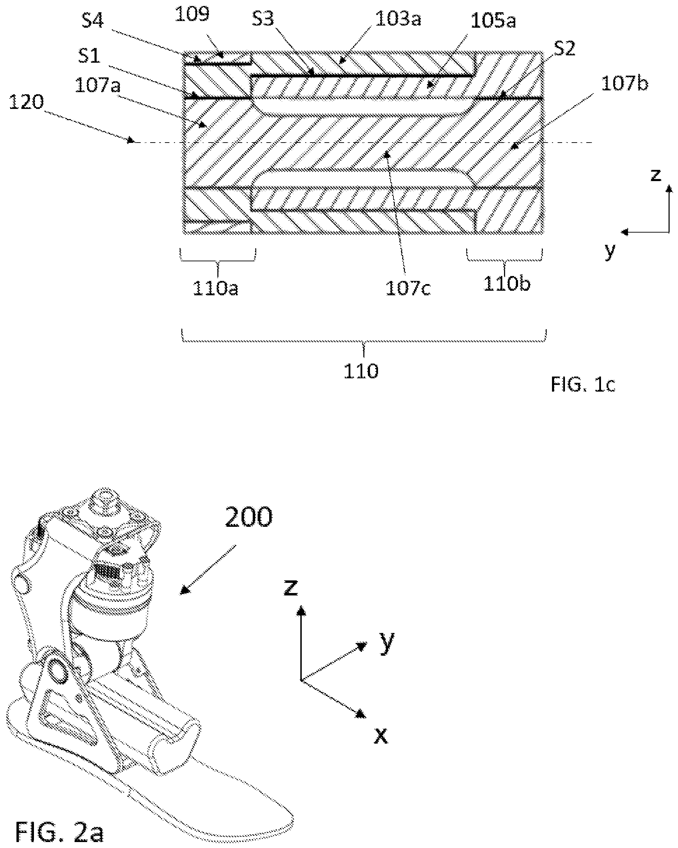

Les figures 1a, 1b, et 1c illustrent schématiquement l'ensemble articulé, suivant une vue en perspective, une vue éclatée, et une vue en coupe.

En référence aux figures 1a à 1c, l'ensemble articulé 101 peut comprendre un élément d'entrée 103, un élément de sortie 105, une barre de torsion 107 apte à se déformer autour d'un axe longitudinal 120 de la barre de torsion et des moyens d'actionnement. L'élément d'entrée 103 peut comporter une section tubulaire à partir de laquelle s'étend une biellette formant par exemple une chape d'articulation. En particulier, l'extrémité libre du bras peut comporter un orifice destiné au montage d'une articulation.

La section tubulaire de l'élément d'entrée 103 peut être couplé en rotation à une première zone 107a de la barre de torsion 107 et l'élément de sortie 105 peut être couplé en rotation à une deuxième zone 107b de la barre de torsion. L'élément d'entrée 103, en particulier le bras, peut être relié aux moyens d'actionnement et l'élément d'entrée et l'élément de sortie 105 peuvent être guidés en rotation l'un par rapport l'autre suivant l'axe 120 de la barre de torsion.

À titre d'exemple, la barre de torsion 107 peut comprendre une première extrémité formant la première zone de la barre de torsion et peut comprendre une deuxième extrémité formant la deuxième zone de la barre de torsion. La première zone et la deuxième zone de la barre de torsion peuvent être cylindriques.

La barre de torsion 107 peut comprendre une portion de travail 107c, par exemple cylindrique, comprise entre la première extrémité et la deuxième extrémité de la barre de torsion 107 et configurée pour se déformer par torsion. La première zone 107a et la deuxième zone 107b de la barre de torsion 107 peuvent présenter une section ou un diamètre plus important que la section ou le diamètre de la portion de travail 107c.

La raideur de la barre de torsion 107 peut être comprise entre 100 et 10 000 N.m par radian, permettant à l'ensemble articulé 101 d'adresser un grand nombre d'applications.

Dans un ou plusieurs modes de réalisation, la portion de travail 107c peut présenter une longueur comprise entre 20 et 70 mm.Le diamètre de la portion de travail est par exemple comprise entre 5 et 15 mm.

En outre, l'élément d'entrée 103 et l'élément de sortie 105 de l'ensemble articulé 101 peuvent comprendre respectivement une première section tubulaire 103a, par exemple cylindrique, et une deuxième section tubulaire 105a, par exemple cylindrique, configurées pour s'emboîter l'une dans l'autre en formant mutuellement un corps tubulaire 110 logeant la barre de torsion 107. Une biellette s'étend depuis la section tubulaire 105a, l'extrémité libre de ladite biellette comportant un orifice destiné au passage d'un axe d'articulation, non représenté.

La première section tubulaire 103a de l'élément d'entrée 103 peut comprendre une première surface S1 de couplage intérieure à une première extrémité 110a du corps tubulaire configurée pour être couplée en rotation avec la première zone 107a de la barre de torsion 107, autour de l'axe 120 de la barre de torsion 107.

De même, la deuxième section tubulaire 105a de l'élément de sortie 105 peut comprendre une deuxième surface S2 de couplage, intérieure à une deuxième extrémité 110b du corps tubulaire 110 configurée pour être couplée en rotation avec la deuxième zone de la barre de torsion 107, autour de l'axe 120 de la barre de torsion 107.

À titre d'exemple, la première surface S1 et la deuxième surface S2 peuvent être couplées respectivement en rotation avec la première zone 107a et la deuxième zone 107b par l'intermédiaire de liaisons encastrement respectives. Les liaisons encastrement peuvent être réalisées par l'intermédiaire de cannelures complémentaires entre la première surface S1 et la première zone 107a ainsi que la deuxième surface S2 et la deuxième zone 107b. Les liaisons encastrement peuvent être réalisées par l'intermédiaire de formes polygonales, par exemple carrées ou hexagonales, complémentaires entre la première surface S1 et la première zone 107a ainsi qu'entre la deuxième surface S2 et la deuxième zone 107b. Un arrêt en translation entre les pièces concernées peut être prévu par tous moyens appropriés, préférentiellement amovibles.

L'utilisation de cannelures et de moyens d'arrêt en translation amovible peut permettre de disposer d'une barre de torsion 107 amovible qui peut être ainsi changée en fonction de son usure, de la physionomie ou la morphologie du patient, ou encore de l'évolution de la physionomie ou de la morphologie du patient.

Plutôt que l'utilisation de cannelures pour les liaisons encastrement, il peut être possible d'utiliser tout autre type de transmission par obstacle entre un arbre et un moyeu, par exemple un assemblage carré, hexagonal, polygonal, goupillé, dentelé, conique ou biconique.

La longueur de chaque section tubulaire 103a, 105a peut être comprise entre 20 et 70 mm. De même, le diamètre de chaque section tubulaire peut être compris entre 10 et 40 mm.

Le corps tubulaire qui s'étend entre la première extrémité 110a et la deuxième extrémité 110b peut loger entièrement la barre de torsion 107. La barre de torsion peut être de même longueur que le corps tubulaire suivant l'axe 120 de la barre de torsion 107.

En outre, la première section tubulaire 103a et la deuxième section tubulaire 105a peuvent comprendre respectivement des premières surfaces de guidage S3, respectivement intérieures et extérieures à la première section tubulaire 103a et à la deuxième section tubulaire 105a, ou inversement. Par exemple, les premières surfaces peuvent comprendre la surface intérieure de la première section tubulaire 103a et la surface extérieure de la deuxième section tubulaire 105a qui sont en contact S3 de manière à assurer un guidage en rotation de l'élément de sortie par rapport à l'élément d'entrée autour de l'axe 120 de la barre de torsion 107.

Dans un ou plusieurs modes de réalisation, inversement, la première section tubulaire 103a peut comprendre une surface extérieure en contact avec une surface intérieure de la deuxième section tubulaire 105a de manière à permettre une rotation guidée de l'élément d'entrée par rapport à l'élément de sortie.

Ces premières surfaces de guidage peuvent être agencées en une position intermédiaire entre la première extrémité 110a et la deuxième extrémité 110b du corps tubulaire 110.

En outre, l'élément de sortie peut comprendre une deuxième partie 109, distincte de la deuxième section tubulaire 105a, comportant une zone annulaire en liaison pivot avec la première section tubulaire 103a de l'élément d'entrée. La liaison pivot peut être réalisée par des deuxièmes surfaces S4 de guidage respectivement extérieures et intérieures à la première section tubulaire 103a et à la deuxième partie, et qui peuvent être agencées à la première extrémité du corps tubulaire. Une biellette s'étend depuis la zone annulaire, l'extrémité libre de la biellette comportant un orifice destiné au passage d'un axe d'articulation, non représenté.

L'élément d'entrée 103, en particulier l'extrémité libre de la biellette de l'élément d'entrée, peut être reliée 115 à des moyens actionnement (non représentés sur les figures 1a-1c) par une liaison pivot.

Les moyens d'actionnement peuvent comprendre un actionneur linéaire configuré pour déplacer un organe d'actionnement apte à être déplacé selon un axe 220 perpendiculaire à l'axe 120 de la barre de torsion 107.

À titre d'exemple, l'actionneur linéaire peut être un vérin à vis, comportant un moteur comprenant un arbre de sortie, l'arbre de sortie étant entraîné en rotation et étant couplé à un réducteur de vitesse, par exemple de type vis-écrou, la vis étant couplée à l'arbre de sortie du moteur, éventuellement par l'intermédiaire d'un système à poulie, ou formant l'arbre de sortie du moteur. La rotation de la vis entraîne le déplacement en translation de l'écrou, le long de l'axe de la vis 203. L'écrou est articulé par rapport à l'extrémité libre de la biellette de l'élément d'entrée, par l'intermédiaire de l'axe XX.

Les différents éléments compris dans l'ensemble articulé peuvent être métalliques.

Un tel ensemble articulé peut être utilisé au sein de dispositif tel qu'un système articulé comprenant un ou plusieurs ensembles articulés, dans des grands appareils orthopédiques (prothèses ou orthèses, par exemple de pied ou de genou), ou encore en robotique, par exemple dans des cobots ou des exosquelettes.

Par exemple, le système articulé peut être un cobot comme un bras articulé utilisé en cobotique, chaque articulation du bras robotique pouvant comprendre un ou plusieurs ensembles articulés agencés en série ou en parallèle selon la présente description.

Selon un autre exemple, un tel ensemble articulé 101 peut être intégré à une prothèse active de pied, l'ensemble articulé 101 remplissant alors la fonction de tendon d'Achille.

Les figures 2a à 2c illustrent schématiquement un appareil prothétique telle qu'une prothèse de pied comprenant l'ensemble articulé de la présente divulgation.

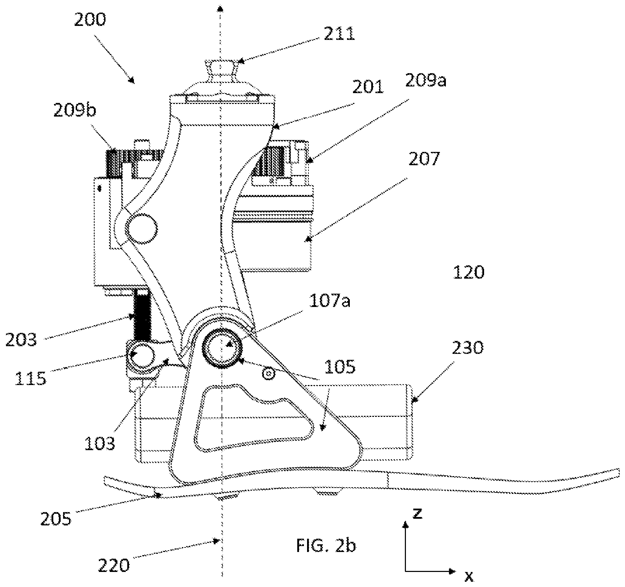

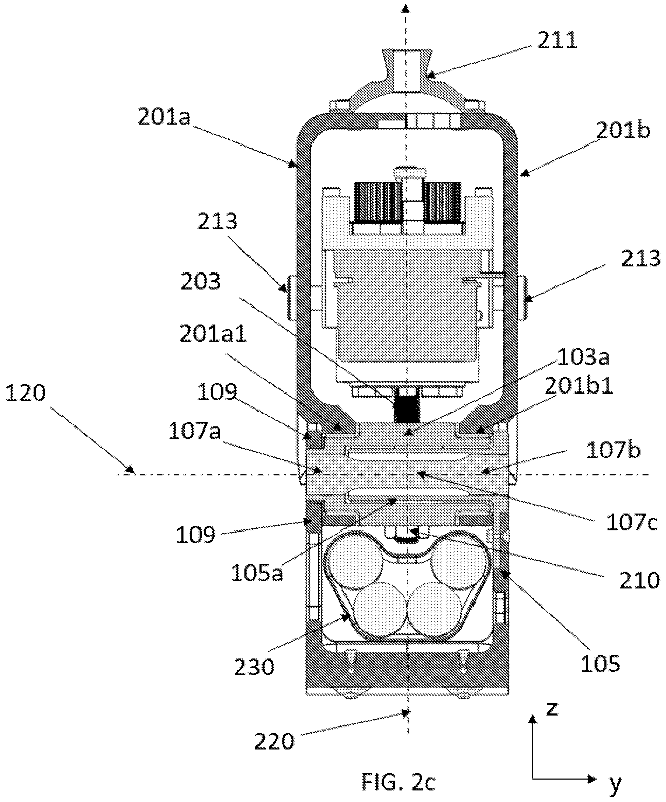

La figure 2a correspond à une vue en perspective, la figure 2b à une vue de côté de la prothèse de pied selon le plan ZX, et la figure 2c illustre une vue en coupe ZY suivant un plan comprenant l'axe de la barre de torsion 107.

En référence aux figures 2a et 2b, la prothèse de pied 200 peut comprendre l'ensemble articulé monté dans une embase tibiale 201 destinée à être fixée à un moignon à travers une emboiture. Par exemple, l'embase tibiale 201 peut se présenter sous la forme d'un support en U inversé comprenant à son sommet une base à partir de laquelle s'étendent vers le bas deux parois latérales. Un adapteur pyramidal 211 s'étend vers le haut depuis la base. Ledit adaptateur présente par exemple une forme de pyramide à base polygonale, en particulier carrée, permettant une fixation sur un moignon d'une personne à travers une emboiture.

Par ailleurs l'élément de sortie 105 peut être relié (e.g. directement ou indirectement) à une lame d'appui au sol 205 (i.e. équivalente à la voute plantaire d'un pied) configurée pour pivoter par rapport à l'embase tibiale autour de l'axe de torsion 120 lors de la marche.

La lame d'appui au sol peut être une lame en matériau composite, par exemple à base de fibres de carbone, de verre, de kevlar

La lame d'appui au sol peut être configurée de sorte à ce que sa flexion participe à la raideur équivalente de l'ensemble du système ramenée à l'axe de la barre de torsion.

Chaque paroi 201a ; 201b de l'embase tibiale peut être montée pivotante sur le corps tubulaire 110 et entre les deux extrémités du corps tubulaire 110a ;110b. Plus précisément, chaque paroi 201a ; 201b peut présenter un orifice dont le bord interne délimite une surface cylindrique guidée en rotation, par l'intermédiaire par exemple de coussinets ou de bagues ou encore de roulements, sur des surfaces cylindriques complémentaires formées par des épaulements de la première section 103a tubulaire de l'élément d'entrée. L'embase est ainsi en liaison pivot avec le corps tubulaire 110 qui comprend l'élément d'entrée, l'élément de sortie et la barre de torsion. Bien évidemment, d'autres types de liaison peuvent être envisagés.

De plus, l'embase tibiale 201 peut délimiter une zone creuse, dans laquelle sont notamment logés les moyens d'actionnement.

Dans un ou plusieurs modes de réalisation, des moyens de contrôle tels qu'une unité de contrôle permettant de piloter les différents éléments de l'appareil prothétique, peuvent également être montés sur l'embase tibiale 201, par exemple dans la zone en creux.

Un tel agencement des moyens d'actionnement et/ou des moyens de contrôle dans l'embase tibiale 201 permet d'augmenter la compacité du pied artificiel, critère important pour tout porteur d'une prothèse active.

Par ailleurs, l'actionneur linéaire peut être un moteur 207 entrainant un système vis-écrou tel que décrit précédemment. Le moteur 207 peut comporter un corps et un arbre de sortie destiné à être entrainé en rotation, et couplé à une poulie menante 209a. Le système vis-écrou comporte également un corps dans lequel l'écrou est monté pivotant, l'écrou étant engagé sur la vis 203. Le corps est articulé sur l'embase tibiale 201, au niveau d'un axe de pivot 213, perpendiculaire à l'axe de la vis, et parallèle à l'axe 115 et à l'axe 107b. Le corps du moteur est fixe par rapport au corps du système vis-écrou. L'écrou est par ailleurs couplé à une poulie menée 209b. La poulie menante 209a est apte à entrainer la poulie menée, par l'intermédiaire d'une courroie par exemple (non représentée).

La rotation de l'arbre du moteur 207 entraîne la rotation de l'écrou d'une vis à rouleaux planétaires (nommée aussi vis à rouleaux satellites) ou d'une vis à billes interne au corps, au travers des poulies 209a, 209b et de la courroie et, ainsi, la translation de la vis le long de l'écrou tournant, provoquant le déplacement par rotation de l'élément d'entrée 103 par rapport à l'élément de sortie 105 au travers de la barre de torsion 107.

Dans un ou plusieurs modes de réalisation, le système à poulie et courroie peut être remplacé par un système d'engrenage, par exemple un engrenage droit, conique, ou encore hélicoïdal. En variante, l'arbre du moteur peut entraîner directement la vis du système vis-écrou. Selon encore une autre variante, l'arbre du moteur peut entraîner directement l'écrou du système vis-écrou.

Dans un ou plusieurs modes de réalisation, le volume total comprenant l'actionneur linéaire et le couple poulie menante/menée peut être compris entre 100 et 1500 cm³ pour une puissance maximale fournie par le moteur comprise entre 100 et 1500 Watts.

Selon un ou plusieurs exemples, la liaison pivot 115 entre l'organe d'actionnement 203 et l'élément d'entrée 103 peut être réalisée à travers d'un palier, tel par exemple qu'un palier lisse ou qu'un roulement par exemple à billes, à rouleaux ou à aiguilles.

Afin de permettre la rotation de l'élément d'entrée 103 par rapport à l'embase tibiale 201 et/ou de permettre la torsion de la barre de torsion 107 lors de la marche d'un porteur de la prothèse de pied, l'actionneur linéaire pivote par rapport à l'embase 201 selon la liaison pivot 213. La torsion de la barre de torsion 107 entraine la rotation de l'actionneur linéaire 207 par rapport à l'embase autour de la liaison pivot 213. La torsion de la barre de torsion 107 permet d'emmagasiner de l'énergie qui sera ensuite délivrée dans la suite du mouvement.

L'alimentation du pied artificiel, en particulier des moyens d'actionnement, peut être effectuée par l'intermédiaire d'une source d'énergie autonome 230 comprise dans la prothèse de pied. Dans un ou plusieurs modes de réalisation, la source d'énergie autonome peut être agencée au sein d'un espace situé entre la barre de torsion 107 et la lame d'appui au sol. Le logement d'une source d'énergie autonome entre la barre de torsion 107 et la lame d'appui est rendu possible par la compacité de l'enchevêtrement de l'ensemble articulé comprenant l'élément d'entrée, la barre de torsion 107 et l'élément de sortie relié à la lame d'appui.

Dans un ou plusieurs modes de réalisation, la source d'énergie autonome 230 peut être une batterie, telle qu'une batterie au lithium.

Dans un ou plusieurs modes de réalisations, le volume total de la batterie peut être compris entre 100 et 300 cm³.

Ainsi, un tel agencement au sein d'une prothèse, telle qu'une prothèse de pied, permet l'obtention d'une prothèse de pied active qui est compacte tout en étant robuste et qui dispose en outre d'une autonomie suffisante pour le quotidien d'un porteur de prothèse de pied.

Dans un tel agencement, la déformation de la barre de torsion 107 intervient lors de la phase d'appui, c'est-à-dire lorsque le tibia passe par-dessus le pied, à l'instar d'un tendon d'Achille. La barre de torsion 107 libère ensuite l'énergie emmagasinée aidant ainsi le moteur à fournir le couple nécessaire à la propulsion de la marche lors de la phase de poussée.

Plus spécifiquement, lors de la phase d'appui par l'intermédiaire de la lame d'appui au sol, la torsion de la barre de torsion 107 par la rotation de l'élément d'entrée par rapport à l'élément de sortie emmagasine de l'énergie. En même temps que la torsion (ou déformation) de la barre de torsion 107, le moteur entraine la vis de manière à permettre la déformation de la barre de torsion 107. Au moment du transfert du poids du porteur de prothèse d'une jambe à l'autre, le moteur tire sur l'élément d'entrée par la montée de la vis de manière à enclencher la propulsion, et du fait du basculement du poids, l'énergie emmagasinée peut être restituée, par relâchement de la barre de torsion 107, pour la propulsion, simultanément avec le travail du moteur.

De manière optionnelle, et dans un ou plusieurs modes de réalisation, au moins un ressort, par exemple un ressort de de traction, peut être disposé entre l'actionneur linéaire et l'embase. Le ressort peut être configuré pour se tendre ou se comprimer lors du mouvement relatif entre l'actionneur linéaire et l'embase.

Par exemple, la rotation de l'embase tibiale 201 autour du point pivot, lors de la marche, peut entrainer la traction d'un ressort situé entre l'embase tibiale 201 et le couple poulies menante/menée. L'utilisation de ce ressort en série avec la barre de torsion 107 permet de faire varier la raideur globale de l'appareil prothétique par ajustement de la raideur du ressort, la raideur globale de l'appareil prothétique étant ramenée au niveau de la barre de torsion 107.

Dans un ou plusieurs modes de réalisation, d'autres configurations du ressort ou de la localisation du ressort peuvent être envisageables.

En outre, de manière générale, dans un ou plusieurs modes de réalisation, il est possible d'améliorer l'irréversibilité de l'ensemble vis-écrou à rouleaux planétaires en modifiant la pente des filets, en rendant le filet asymétrique, en alternant filets asymétriques et symétriques entre vis, rouleaux et écrous ou encore en ajoutant des joints augmentant la friction.

## Revendications

1. Ensemble articulé (101) comprenant :
- un élément d'entrée (103),
- un élément de sortie (105 ;109),
- des moyens d'actionnement, l'élément d'entrée (103) étant en outre reliée aux moyens d'actionnement, l'élément d'entrée (103) et l'élément de sortie (105; 109) étant guidés en rotation l'un par rapport l'autre;
**caractérisé en ce que** l'ensemble articulé comprend en outre une barre de torsion (107) apte à se déformer autour d'un axe (120) longitudinal de la barre de torsion (107),
dans lequel l'élément d'entrée (103) est couplé en rotation à une première zone (107a) de la barre de torsion (107) et l'élément de sortie (105) est couplé en rotation à une deuxième zone (107b) de la barre de torsion (107), l'élément d'entrée (103) et l'élément de sortie (105) étant guidés en rotation l'un par rapport l'autre suivant l'axe (120) de la barre de torsion (107).

2. Ensemble articulé selon la revendication précédente, dans lequel l'élément d'entrée (103) et l'élément de sortie (105) sont reliés respectivement à la première zone et à la deuxième zone de la barre de torsion (107) par l'intermédiaire de liaisons d'encastrement.

3. Ensemble articulé selon l'une quelconque des revendications précédentes dans lequel la barre de torsion (107) présente une valeur de raideur comprise entre 100 et 10 000 N.m par radian.

4. Ensemble articulé selon l'une quelconque des revendications précédentes dans lequel la barre de torsion (107), l'élément d'entrée (103) et l'élément de sortie (105) sont configurés de manière à ce que la barre de torsion (107) soit amovible.

5. Ensemble articulé selon l'une quelconque des revendications précédentes dans lequel l'ensemble comprend une embase articulée par rapport à l'élément d'entrée (103), l'embase supportant les moyens d'actionnement.

6. Ensemble articulé selon l'une quelconque des revendications précédentes dans lequel les moyens d'actionnement comprennent un actionneur linéaire configuré pour déplacer un organe d'actionnement apte à être déplacé selon un axe d'actionnement non colinéaire, par exemple perpendiculaire, à l'axe de la barre de torsion (107), le déplacement de l'organe d'actionnement entrainant la rotation de l'élément d'entrée (103) par rapport à l'élément de sortie (105).

7. Ensemble articulé selon la revendication précédente dans lequel l'organe d'actionnement est en liaison pivot ou rotule avec l'élément d'entrée (103).

8. Ensemble articulé selon la revendication précédente dans lequel l' élément d'entrée (103) est relié à l'embase [pièce tibiale] par une liaison pivot.

9. Ensemble selon la combinaison des revendications 5 à 8 dans lequel l'actionneur linéaire est articulé à l'embase selon une liaison pivot dont l'axe de pivot est parallèle à l'axe de la barre de torsion (107) et dans lequel la torsion de la barre de torsion (107) ou la rotation de l'élément d'entrée (103) entraine la rotation de l'actionneur linéaire par rapport à l'embase autour du deuxième axe de pivot.

10. Ensemble selon la revendication 9, comprenant au moins un ressort entre l'actionneur linéaire et l'embase, ledit au moins un ressort étant configuré pour se comprimer ou se détendre lors du mouvement relatif entre l'actionneur linéaire et ladite embase.

11. Ensemble articulé selon la revendication précédente, dans lequel l'au moins un ressort est configuré de façon à ce que la raideur globale est comprise entre 100 et 10 000 N.m par radian.

12. Ensemble articulé selon l'une quelconque des revendications 2 à 11, dans lequel une première extrémité de la barre de torsion (107) forme la première zone (107a) et une deuxième extrémité de la barre de torsion (107) forme la deuxième zone (107b), ladite barre de torsion (107) étant configurée pour se déformer par torsion dans une portion de travail (107c) comprise entre la première zone et la deuxième zone,
l'élément d'entrée (103) et l'élément de sortie (105) comprenant respectivement une première section tubulaire (103a) et une deuxième section tubulaire (105a) configurées pour s'emboîter l'une dans l'autre en formant mutuellement un corps tubulaire (110) logeant la barre de torsion (107), la première section tubulaire (103a) de l'élément d'entrée (103) comprenant une première surface de couplage intérieure à une première extrémité du corps tubulaire configurée pour être couplée en rotation avec la première zone de la barre de torsion (107) autour de l'axe de la barre de torsion (107) et la deuxième section tubulaire (105a) de l'élément de sortie (105) comprenant une deuxième surface de couplage intérieure à une deuxième extrémité du corps tubulaire configurée pour être couplée en rotation avec la deuxième zone de la barre de torsion (107), autour de l'axe de la barre de torsion (107),
et dans lequel la première section tubulaire (103a) et la deuxième section tubulaire (105a) comprennent respectivement des premières surfaces de guidage, respectivement intérieures et extérieures à la première section tubulaire (103a) et à la deuxième section tubulaire (105a), ou inversement , les première surfaces de guidage agencées en une position intermédiaire entre la première extrémité et la deuxième extrémité du corps tubulaire, les premières surfaces de guidage intérieures et extérieures configurées pour assurer un guidage en rotation de l'élément de sortie (105) par rapport à l'élément d'entrée (103) autour de l'axe de la barre de torsion (107).

13. Ensemble articulé selon la revendication 12 dans lequel l'élément de sortie (105) comprend une deuxième partie, distincte de la deuxième section tubulaire (105a), en liaison pivot avec la première section tubulaire (103a) de l'élément d'entrée (103), par des deuxièmes surfaces de guidage respectivement extérieures et intérieures à la première section tubulaire 103a et à la deuxième partie, agencées à la première extrémité du corps tubulaire

14. Ensemble articulé selon la revendication précédente dans lequel le corps tubulaire s'étendant entre la première extrémité et la deuxième extrémité loge entièrement la barre de torsion (107), la barre de torsion (107) étant de préférence de même longueur que le corps tubulaire suivant l'axe de la barre de torsion (107).

15. Appareil prothétique comprenant un ensemble articulé selon la revendication 5 seule ou en combinaison avec l'une quelconque 2 à 4 et 6 à 14 comprenant :
- l'embase est une embase tibiale destinée à être fixée à un moignon, l'embase tibiale embarquant les moyens d'actionnement,
- une lame d'appui au sol configurée pour pivoter par rapport à l'embase autour de l'axe de la barre de torsion (107),
et dans lequel l'élément d'entrée (103) est connecté à une sortie des moyens d'actionnement et l'élément de sortie 105 est relié à la lame d'appui au sol,
la barre de torsion (107) étant configurée, lors de la marche, pour emmagasiner une énergie par torsion de la barre de torsion (107), et de la restituer pour la propulsion simultanément au travail des moyens d'actionnement.

16. Appareil prothétique selon la revendication 15, dans l'embase tibiale comprend un corps creux recevant les moyens d'actionnement.

17. Appareil prothétique selon la revendication 16, l'embase tibiale présente une direction longitudinale perpendiculairement à la barre de torsion (107), les moyens d'actionnement comprennent un moteur et un système vis-écrou, le système vis-écrou et le moteur étant agencés en recouvrement suivant l'axe longitudinal.

18. Appareil prothétique selon l'une des revendications 15 à 17, dans lequel un espace est présent entre la barre de torsion (107) et la plaque d'appui recevant une source d'énergie autonome pour les moyens d'actionnement.

19. Grand appareil orthopédique comportant au moins un ensemble articulé selon l'une quelconque des revendications 1 à 14.

20. Système articulé comprenant au moins un ensemble articulé selon l'une quelconque des revendications 1 à 14, dans lequel le système articulé comprend au moins une première section et une deuxième section articulée l'une par rapport l'autre, la première section formant l'élément d'entrée (103) de l'ensemble d'articulé, la deuxième section formant l'élément de sortie (105) de l'ensemble d'articulé.

## Patentansprüche

1. Gelenkanordnung (101), umfassend:
- ein Eingangselement (103),
- ein Ausgangselement (105; 109),
- Betätigungsmittel, wobei ferner das Eingangselement (103) mit den Betätigungsmitteln verbunden ist, wobei das Eingangselement (103) und das Ausgangselement (105; 109) relativ zueinander drehbar geführt sind;
**dadurch gekennzeichnet, dass** die Gelenkanordnung ferner einen Torsionsstab (107) umfasst, der sich um eine Längsachse (120) des Torsionsstabs (107) verformen kann,
wobei das Eingangselement (103) drehbar mit einem ersten Bereich (107a) des Torsionsstabs (107) gekoppelt ist und das Ausgangselement (105) drehbar mit einem zweiten Bereich (107b) des Torsionsstabs (107) gekoppelt ist,
wobei das Eingangselement (103) und das Ausgangselement (105) relativ zueinander drehbar entlang der Achse (120) des Torsionsstabs (107) geführt sind.

2. Gelenkanordnung nach dem vorhergehenden Anspruch, wobei das Eingangselement (103) und das Ausgangselement (105) jeweils über Einsteckverbindungen mit dem ersten Bereich bzw. dem zweiten Bereich des Torsionsstabes (107) verbunden sind.

3. Gelenkanordnung nach einem der vorhergehenden Ansprüche,
wobei der Torsionsstab (107) einen Steifigkeitswert zwischen 100 und 10.000 N.m pro Radiant aufweist.

4. Gelenkanordnung nach einem der vorhergehenden Ansprüche,
wobei der Torsionsstab (107), das Eingangselement (103) und das Ausgangselement (105) so ausgelegt sind, dass der Torsionsstab (107) abnehmbar ist.

5. Gelenkanordnung nach einem der vorhergehenden Ansprüche,
wobei die Anordnung eine Basis umfasst, die in Bezug auf das Eingangselement (103) gelenkig ausgebildet ist, wobei die Basis die Betätigungsmittel trägt.

6. Gelenkanordnung nach einem der vorhergehenden Ansprüche,
wobei die Betätigungsmittel einen Linearaktuator umfassen, der dazu ausgelegt ist, ein Betätigungsorgan zu verschieben, das entlang einer Betätigungsachse verschiebbar ist, die nicht kollinear, beispielsweise senkrecht, zur Achse des Torsionsstabs (107) verläuft, wobei die Verschiebung des Betätigungsorgans eine Drehung des Eingangselements (103) in Bezug auf das Ausgangselement (105) bewirkt.

7. Gelenkanordnung nach dem vorhergehenden Anspruch, wobei das Betätigungsorgan mit dem Eingangselement (103) in einer Schwenk- oder Kugelgelenkverbindung steht.

8. Gelenkanordnung nach dem vorhergehenden Anspruch, wobei das Eingangselement (103) durch eine Schwenkverbindung mit der Basis [Tibia-Teil] verbunden ist.

9. Anordnung nach den Ansprüchen 5 bis 8 in Kombination, wobei der Linearaktuator an der Basis über eine Schwenkverbindung angelenkt ist, deren Schwenkachse parallel zur Achse des Torsionsstabs (107) verläuft, und wobei die Torsion des Torsionsstabs (107) oder die Drehung des Eingabeelements (103) eine Drehung des Linearaktuators relativ zur Basis um die zweite Schwenkachse bewirkt.

10. Anordnung nach Anspruch 9, umfassend zumindest eine Feder zwischen dem Linearaktuator und der Basis, wobei die zumindest eine Feder dazu ausgelegt ist, bei der Relativbewegung zwischen dem Linearaktuator und der Basis komprimiert oder entspannt zu werden.

11. Gelenkanordnung nach dem vorhergehenden Anspruch, wobei die zumindest eine Feder so ausgelegt ist, dass die Gesamtsteifigkeit zwischen 100 und 10.000 N.m pro Radiant liegt.

12. Gelenkanordnung nach einem der Ansprüche 2 bis 11, wobei ein erstes Ende des Torsionsstabs (107) den ersten Bereich (107a) bildet und ein zweites Ende des Torsionsstabs (107) den zweiten Bereich (107b) bildet, wobei der Torsionsstab (107) dazu ausgelegt ist, sich in einem Arbeitsabschnitt (107c) zwischen dem ersten Bereich und dem zweiten Bereich durch Torsion zu verformen,
wobei das Eingangselement (103) und das Ausgangselement (105) jeweils einen ersten röhrenförmigen Abschnitt (103a) bzw. einen zweiten röhrenförmigen Abschnitt (105a) umfassen, die dazu ausgelegt sind, ineinanderzugreifen, um zusammen einen röhrenförmigen Körper (110) zu bilden, der den Torsionsstab (107) aufnimmt, wobei der erste röhrenförmige Abschnitt (103a) des Eingangselements (103) eine erste innere Kopplungsfläche an einem ersten Ende des röhrenförmigen Körpers umfasst, die dazu ausgelegt ist, mit dem ersten Bereich des Torsionsstabs (107) um die Achse des Torsionsstabs (107) drehbar gekoppelt zu werden, und der zweite röhrenförmige Abschnitt (105a) des Ausgangselements (105) eine zweite innere Kopplungsfläche an einem zweiten Ende des röhrenförmigen Körpers umfasst, die dazu ausgelegt ist, mit dem zweiten Bereich des Torsionsstabs (107) um die Achse des Torsionsstabes (107) drehbar gekoppelt zu werden,
und wobei der erste rohrförmige Abschnitt (103a) und der zweite rohrförmige Abschnitt (105a) jeweils erste Führungsflächen umfassen, die jeweils innen bzw. außen an dem ersten rohrförmigen Abschnitt (103a) bzw. dem zweiten rohrförmigen Abschnitt (105a) liegen, oder umgekehrt, wobei die ersten Führungsflächen an einer Zwischenposition zwischen dem ersten Ende und dem zweiten Ende des rohrförmigen Körpers angeordnet sind, wobei die inneren und äußeren ersten Führungsflächen dazu ausgelegt sind, eine Drehführung des Ausgangselements (105) relativ zu dem Eingangselement (103) um die Achse des Torsionsstabes (107) sicherzustellen.

13. Gelenkanordnung nach Anspruch 12,
wobei das Ausgangselement (105) ein zweites Teil umfasst, das sich von dem zweiten rohrförmigen Abschnitt (105a) unterscheidet und mit dem ersten rohrförmigen Abschnitt (103a) des Eingangselements (103) über zweite Führungsflächen, die jeweils außen bzw. innen an dem ersten rohrförmigen Abschnitt 103a bzw. dem zweiten Teil liegen und an dem ersten Ende des rohrförmigen Körpers angeordnet sind, schwenkbar verbunden ist.

14. Gelenkanordnung nach dem vorhergehenden Anspruch, wobei der rohrförmige Körper, der sich zwischen dem ersten Ende und dem zweiten Ende erstreckt, den Torsionsstab (107) vollständig aufnimmt, wobei der Torsionsstab (107) vorzugsweise die gleiche Länge wie der rohrförmige Körper entlang der Achse des Torsionsstabs (107) hat.

15. Protheseneinrichtung mit einer Gelenkanordnung nach Anspruch 5 allein oder in Kombination mit einem der Ansprüche 2 bis 4 und 6 bis 14, umfassend:
- die Basis, bei der es sich um eine Tibia-Basis handelt, die an einem Stumpf befestigt werden soll, wobei die Tibia-Basis die Betätigungsmittel mitführt,
- eine Bodenaufstandsplatte, die dazu ausgelegt ist, relativ zur Basis um die Achse des Torsionsstabs (107) zu schwenken,
und wobei das Eingangselement (103) mit einem Ausgang der Betätigungsmittel verbunden ist und das Ausgangselement (105) mit der Bodenaufstandsplatte verbunden ist,
wobei der Torsionsstab (107) dazu ausgelegt ist, beim Gehen durch Verdrehen des Torsionsstabs (107) Energie zu speichern und diese für den Antrieb gleichzeitig mit der Betrieb der Betätigungsmittel wieder abzugeben.

16. Protheseneinrichtung nach Anspruch 15,
wobei die Tibia-Basis einen Hohlkörper umfasst, der die Betätigungsmittel aufnimmt.

17. Protheseneinrichtung nach Anspruch 16,
wobei die Tibia-Basis eine Längsrichtung senkrecht zum Torsionsstab (107) aufweist, wobei die Betätigungsmittel einen Motor und ein Schrauben-Mutter-System umfassen, wobei das Schrauben-Mutter-System und der Motor entlang der Längsachse überlappend angeordnet sind.

18. Protheseneinrichtung nach einem der Ansprüche 15 bis 17,
wobei zwischen dem Torsionsstab (107) und der eine autonome Energiequelle für die Betätigungsmittel aufnehmenden Aufstandsplatte ein Zwischenraum vorhanden ist.

19. Ortheseneinrichtung mit zumindest einer Gelenkanordnung nach einem der Ansprüche 1 bis 14.

20. Gelenksystem mit zumindest einer Gelenkanordnung nach einem der Ansprüche 1 bis 14, wobei das Gelenksystem zumindest einen ersten Abschnitt und einen zweiten Abschnitt aufweist, die gelenkig zueinander ausgebildet sind, wobei der erste Abschnitt das Eingangselement (103) der Gelenkanordnung bildet und der zweite Abschnitt das Ausgangselement (105) der Gelenkanordnung bildet.

## Claims

1. Articulated assembly (101) comprising:
- an input element (103),
- an output element (105;109),
- actuation means, the input element (103) further being connected to the actuation means, the input element (103) and the output element (105) being guided in rotation relative to one another,
**characterized in that** the articulated assembly comprises a torsion bar (107) capable of deforming about a longitudinal axis (120) of the torsion bar (107),
wherein the input element (103) is rotationally coupled to a first zone (107a) of the torsion bar (107) and the output element (105) is rotationally coupled to a second zone (107b) of the torsion bar (107), the input element (103) and the output element (105) being guided in rotation relative to one another along the axis (120) of the torsion bar (107).

2. Articulated assembly according to the preceding claim, wherein the input element (103) and the output element (105) are respectively connected to the first zone and to the second zone of the torsion bar (107) via embedding connections.

3. Articulated assembly according to any one of the preceding claims, wherein the torsion bar (107) has a stiffness value of between 100 and 10,000 N.m per radian.

4. Articulated assembly according to any one of the preceding claims, wherein the torsion bar (107), the input element (103), and the output element (105) are configured so that the torsion bar (107) is removable.

5. Articulated assembly according to any one of the preceding claims, wherein the assembly comprises a base that is hinged relative to the input element (103), the base supporting the actuation means.

6. Articulated assembly according to any one of the preceding claims, wherein the actuation means comprise a linear actuator configured to move an actuating member capable of being moved along an actuation axis that is non-collinear, for example perpendicular, to the axis of the torsion bar (107), the movement of the actuating member causing rotation of the input element (103) relative to the output element (105).

7. Articulated assembly according to the preceding claim, wherein the actuating member is in a pivoting or ball joint connection with the input element (103).

8. Articulated assembly according to the preceding claim, wherein the input element (103) is connected to the base by a pivoting connection.

9. Assembly according to the combination of claims 5 to 8, wherein the linear actuator is hinged to the base in a pivoting connection in which the pivot axis is parallel to the axis of the torsion bar (107) and in which the torsion of the torsion bar (107) or the rotation of the input element (103) causes rotation of the linear actuator relative to the base about the second pivot axis.

10. Assembly according to claim 9, comprising at least one spring between the linear actuator and the base, said at least one spring being configured to compress or relax during relative movement between the linear actuator and said base.

11. Articulated assembly according to the preceding claim, wherein the at least one spring is configured so that the overall stiffness is between 100 and 10,000 N.m per radian.

12. Articulated assembly according to any one of claims 2 to 11, wherein a first end of the torsion bar (107) forms the first zone (107a) and a second end of the torsion bar (107) forms the second zone (107b), said torsion bar (107) being configured to deform by torsion in a working portion (107c) that is between the first zone and the second zone,
the input element (103) and the output element (105) respectively comprising a first tubular section (103a) and a second tubular section (105a) which are configured to fit into each other, together forming a tubular body (110) housing the torsion bar (107), the first tubular section (103a) of the input element (103) comprising a first internal coupling surface at a first end of the tubular body, configured to be rotationally coupled with the first zone of the torsion bar (107) about the axis of the torsion bar (107), and the second tubular section (105a) of the output element (105) comprising a second internal coupling surface at a second end of the tubular body, configured to be rotationally coupled with the second zone of the torsion bar (107) about the axis of the torsion bar (107),
and wherein the first tubular section (103a) and the second tubular section (105a) respectively comprise first guide surfaces, respectively internal and external to the first tubular section (103a) and to the second tubular section (105a), or vice versa, the first guide surfaces arranged in an intermediate position between the first end and the second end of the tubular body, the internal and external first guide surfaces configured to ensure guidance in rotation of the output element (105) relative to the input element (103) about the axis of the torsion bar (107).

13. Articulated assembly according to claim 12, wherein the output element (105) comprises a second portion, distinct from the second tubular section (105a), in pivoting connection with the first tubular section (103a) of the input element (103), via second guide surfaces which are respectively external and internal to the first tubular section 103a and to the second portion, arranged at the first end of the tubular body.

14. Articulated assembly according to the preceding claim, wherein the tubular body extending between the first end and the second end entirely houses the torsion bar (107), the torsion bar (107) preferably being of the same length as the tubular body along the axis of the torsion bar (107).

15. Prosthetic device comprising an articulated assembly according to claim 5 alone or in combination with any of claims 2 to 4 and 6 to 14, comprising:
- the base is a tibial base intended to be fixed to a limb stump, the tibial base carrying the actuation means,
- a ground contact blade configured to pivot relative to the base about the axis of the torsion bar (107),
and wherein the input element (103) is connected to an output of the actuation means and the output element 105 is connected to the ground contact blade,
the torsion bar (107) being configured to store energy during walking by the torsion of the torsion bar (107), and to supply energy for propulsion simultaneously with the work from the actuation means.

16. Prosthetic device according to claim 15, wherein the tibial base comprises a hollow body receiving the actuation means.

17. Prosthetic device according to claim 16, wherein the tibial base has a longitudinal direction perpendicular to the torsion bar (107), and the actuation means comprise a motor and a screw-nut system, the screw-nut system and the motor being arranged so that they overlap along the longitudinal axis.

18. Prosthetic device according to one of claims 15 to 17, wherein a space is present between the torsion bar (107) and the contact plate, for receiving an independent energy source for the actuation means.

19. Large orthopedic device comprising at least one articulated assembly according to any one of claims 1 to 14.

20. Articulated system comprising at least one articulated assembly according to any one of claims 1 to 14, wherein the articulated system comprises at least a first section and a second section which are hinged relative to one another, the first section forming the input element (103) of the articulated assembly, the second section forming the output element (105) of the articulated assembly.
